Europäisches Patentamt

European Patent Office

Office européen des brevets

(19)

(11) EP 0 558 409 B1

(12) FASCICULE DE BREVET EUROPEEN

(45) Date de publication et mention
de la délivrance du brevet:
19.06.1996 Bulletin 1996/25

(51) Int Cl.⁶: **H02M 3/157**, G05F 1/46,
A61N 1/30, A61N 1/372

(21) Numéro de dépôt: 93400475.5

(22) Date de dépôt: 24.02.1993

(54) **Dispositif de génération d'une tension électrique de forme d'onde prédéterminée et appareil ionophorétique d'administration transdermique de médicaments muni d'un tel dispositif**

Vorrichtung zum generieren einer elektrischen Spannung mit vorgebbaren Wellenform sowie damit versehene Einrichtung zur iontophoretischen transdermalen Abgabe von Medikamenten

Device for generating an electric voltage having a predetermined waveform and iontophoretic transermal drug delivery apparatus, equipped with such a device

(84) Etats contractants désignés:
AT BE CH DE DK ES FR GB GR IE IT LI LU MC NL PT SE

(30) Priorité: 27.02.1992 FR 9202276

(43) Date de publication de la demande:
01.09.1993 Bulletin 1993/35

(73) Titulaire: **LABORATOIRES D'HYGIENE ET DE DIETETIQUE L.H.D. Société Anonyme dite:**
F-75008 Paris (FR)

(72) Inventeurs:
• **Teillaud, Eric**
F-21240 Talant (FR)

• **Bevan, Bruno**
F-21800 Chevigny St Sauveur (FR)
• **Mikler, Claude**
F-21000 Dijon (FR)
• **Reilly, Paul**
Hardwick, Cambs CB3 7QJ (GB)

(74) Mandataire: **Colas, Jean-Pierre et al Cabinet de Boisse**
37, avenue Franklin D. Roosevelt
75008 Paris (FR)

(56) Documents cités:
WO-A-88/08729     DE-A- 3 334 461
FR-A- 2 603 755     FR-A- 2 656 223
US-A- 3 970 919

## Description

La présente invention est relative à un dispositif de génération d'une tension électrique de forme d'onde prédéterminée et, plus particulièrement, à un appareil ionophorétique d'administration transdermique de médicaments muni d'un tel dispositif.

On a proposé des appareils d'administration de médicaments par ionophorèse conçus pour faire pénétrer des molécules ionisées d'un principe actif, que l'on appellera dans la suite "médicament", à travers la peau d'un patient, la circulation des molécules ionisées étant forcée par une différence de potentiel établie entre deux plages adjacentes de la peau du patient. A titre d'exemple de tels appareils, on peut citer ceux décrits dans les documents WO-A-88/08729 et FR-A-2 656 223.

La quantité de médicaments ainsi absorbée par le patient par unité de temps est clairement fonction du courant électrique créé par la différence de potentiel appliquée et qui traverse la peau du patient en passant d'une plage à l'autre. Des études ont montré l'intérêt d'un contrôle précis de l'intensité de ce courant, qui peut être établi ou coupé pendant des périodes de temps successives prédéterminées, ou modulé en intensité suivant des impulsions de forme d'onde sinusoïdale, triangulaire, en dents de scie ou en créneaux, suivant divers programmes d'administration du médicament adaptés à des traitements particuliers, à des molécules particulières, etc... On pourra se référer à cet égard à l'article intitulé "Study of the mechanisms of flux enhancement through hairless mouse skin by pulsed DC iontophoresis" par Pikal et Shah, publié dans la revue "Pharmaceutical Research" volume 8, No. 3, 1991 pages 365 à 369 et à l'article intitulé "Facilitated transdermal delivery of therapeutic peptides and proteins by iontophoretic delivery devices" par Chien et autres, publié dans la revue "Journal of Controlled Release", 13, 1990 pages 263 à 278. Comme on l'explique dans ces publications, l'application prolongée d'une tension continue à la peau provoque une polarisation électrochimique de celle-ci du fait notamment d'un effet capacitif observé dans la couche cornée, cette polarisation provoquant une décroissance du courant électrique associé au transfert ionophorétique des molécules ionisées constituant le médicament, et donc une diminution des quantités de médicament administrées par unité de temps, par rapport aux quantités souhaitées. Une solution connue à ce problème consiste à utiliser des tensions continues pulsées ménageant des temps de dépolarisation de la couche cornée.

Pour l'administration transdermique de médicaments assistée par ionophorèse, on cherche actuellement à réaliser des appareils d'administration prenant la forme de bracelets légers et peu encombrants pouvant être fixés sans gêne sur un bras du patient, pendant plusieurs heures par exemple. De tels bracelets doivent comprendre une réserve de médicaments à administrer et des moyens de génération d'une tension électrique propre à assurer une assistance ionophorétique de la migration des molécules ionisées constituant le médicament, à travers la peau du patient. La réserve de médicaments peut être constituée par un hydrogel chargé de la forme ionisée du médicament et placé entre la peau du patient et l'une au moins des deux électrodes entre lesquelles on applique une tension de forme d'onde prédéterminée. Les moyens de génération de cette tension doivent idéalement, comme on l'a vu plus haut, être capables de délivrer des tensions de forme d'onde, de fréquence et d'amplitude très variées, ceci de manière programmable pour donner à l'appareil une grande souplesse d'utilisation. L'alimentation électrique de l'appareil doit préférentiellement être établie par des piles pour assurer l'autonomie du patient, ce qui oblige à tenir compte de la durée de vie limitée de celles-ci et à prévoir des moyens d'élévation de la tension délivrée par les piles, l'administration transdermique et ionophorétique de médicaments exigeant couramment des tensions instantanées de 10 V ou plus. Ces contraintes, combinées à celles de faible encombrement et de faible poids rendent la conception de tels appareils difficile.

En particulier, pour constituer des moyens de génération de tension programmables en forme d'onde, on peut penser à utiliser un convertisseur numérique-analogique dont l'entrée numérique est alimentée par un dispositif de commande tel qu'un microprocesseur, ce dernier fournissant successivement au convertisseur des valeurs numériques images de l'amplitude de segments successifs d'une forme d'onde de tension quelconque. Malheureusement, compte tenu de la forte tension de sortie nécessaire évoquée ci-dessus, cette solution conduit à utiliser un convertisseur sous boîtier séparé, ce qui accroît le coût et l'encombrement de l'appareil. En outre, un convertisseur numérique-analogique est assimilable à un amplificateur de classe B, c'est-à-dire un amplificateur agissant sur une demi-période de la forme d'onde seulement et présentant donc un rendement sensiblement inférieur à 100 %.

Du document DE-A- 3 334 461, il est connu d'utiliser des moyens de mémoire et un compteur afin de controler la forme de la tension de sortie d'un onduleur commandé en modulation de largeur d'impulsion.

La présente invention a donc pour but de réaliser un dispositif de génération d'une tension électrique de forme d'onde prédéterminée, utilisable dans un appareil d'administration transdermique de médicaments assisté par ionophorèse, qui ne présente pas les inconvénients des solutions connues évoquées ci-dessus, qui soit en particulier léger, peu encombrant et alimenté par des piles électriques de manière à être autonome et portable sans gêne par le patient pendant de longues périodes de temps.

La présente invention a aussi pour but de réaliser un tel dispositif et un tel appareil autorisant une programmation complète de la forme d'onde de la tension appliquée et du programme d'administration du médicament, de manière à donner à l'appareil une grande souplesse d'utilisation.

La présente invention a encore pour but de réaliser un tel dispositif et un tel appareil qui autorisent la génération d'impulsions de forme d'onde de tension alternative, pour assurer notamment des migrations du médicament dans les deux sens successifs du courant ainsi établi.

La présente invention a en outre pour but de réaliser un tel dispositif et un tel appareil qui permettent d'assurer une régulation du courant ou de la tension appliqué de manière à compenser des variations temporelles de la résistance du patient, telle qu'elle est vue par l'appareil entre deux électrodes d'application de la tension qu'il développe.

On atteint ces buts de l'invention, ainsi que d'autres qui apparaîtront à la lecture de la description qui va suivre, avec un dispositif de génération d'une tension électrique de forme d'onde prédéterminée, comprenant une alimentation à découpage munie d'un organe de commutation électronique dont la fermeture commande l'alimentation d'une inductance qui se décharge, à la réouverture de l'organe, dans une capacité aux bornes de laquelle apparaît la tension de sortie du dispositif. Suivant l'invention, celui-ci comprend a) des moyens de mémoire pour enregistrer une suite de nombres qui sont des images de segments successifs de la forme d'onde prédéterminée, b) un compteur numérique cadencé et c) des moyens pour charger successivement ce compteur avec chacun des nombres de la suite en tant que borne du comptage opéré par le compteur, celui-ci commandant cycliquement la fermeture de l'organe de commutation pendant un intervalle de temps prédéterminé, à chaque fois que le comptage opéré atteint la borne ainsi fixée.

Grâce à l'utilisation suivant l'invention d'une alimentation à découpage commandée par un compteur, on obtient un dispositif compact, léger et autonome, offrant une grande souplesse d'utilisation en matière de forme d'onde disponible.

Suivant un mode de réalisation préféré du dispositif selon l'invention, le compteur est un compteur régressif décomptant à partir du nombre chargé et muni d'une sortie de détection de zéro commandant la fermeture de l'organe de commutation pendant l'intervalle de temps prédéterminé, cet organe étant constitué par un transistor, la sortie de détection de zéro du compteur étant bouclé sur une entrée de validation du compteur pour déclencher un nouveau décomptage à partir du nombre chargé.

Suivant une caractéristique avantageuse du dispositif selon l'invention, celui-ci comprend en outre des moyens de stabilisation du courant, interposés entre la capacité de l'alimentation à découpage et une charge extérieure alimentée par la tension délivrée par le dispositif. Cette disposition permet de rendre le courant circulant dans le patient sensiblement indépendant de la résistance de celui-ci, ce qui est important pour contrôler la quantité de médicament administrée par unité de temps.

Avantageusement, le dispositif comprend en outre des moyens pour inverser le sens de circulation du courant dans une charge extérieure, ces moyens étant activés à la détection du chargement dans le compteur d'un nombre image d'un passage par zéro de la forme d'onde souhaitée de la tension de sortie de ce dispositif, celle-ci étant une forme d'onde alternative à demi-ondes symétriques dont seuls les nombres images d'une demi-onde sont chargés dans les moyens de mémoire.

Suivant une autre caractéristique avantageuse du dispositif selon l'invention, celui-ci comprend en outre des moyens de régulation en boucle fermée de la tension ou de l'intensité du courant de sortie du dispositif.

L'invention permet ainsi de constituer un appareil d'administration transdermique de médicaments, du type comprenant au moins deux électrodes mises en contact avec deux plages voisines de la peau d'un patient, une réserve d'un médicament à molécules véhiculées électriquement étant disposée contre au moins une des électrodes pour venir en contact avec la peau du patient, ces électrodes étant alimentées par la sortie du dispositif de génération de tension électrique suivant la présente invention. Avantageusement une réserve de médicaments est disposée contre chacune des deux électrodes pour permettre une administration du médicament dans les deux sens de circulation du courant entre ces électrodes.

D'autres caractéristiques et avantages de la présente invention apparaîtront à la lecture de la description qui va suivre et à l'examen du dessin annexé dans lequel :

- la figure 1 représente schématiquement le dispositif de génération de tension suivant l'invention,
- la figure 2 est un schéma d'un mode de réalisation particulier des liaisons établies entre un microprocesseur et un compteur formant partie du dispositif suivant l'invention,
- la figure 3 représente en plus de détail la partie du dispositif suivant l'invention qui est commandée par la sortie du détecteur de zéro du compteur de la figure 2 pour alimenter les électrodes d'un appareil ionophorétique d'application transdermique de médicaments suivant l'invention, et
- la figure 4 explique, à titre d'exemple, la génération d'une tension sinusoïdale par le dispositif suivant l'invention.

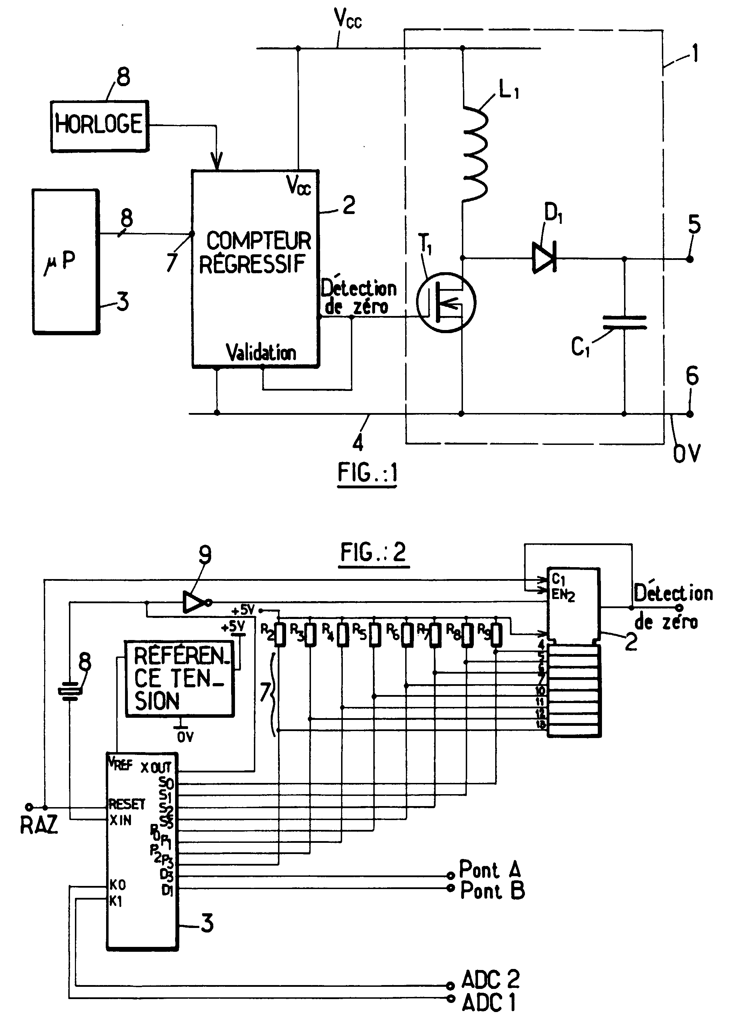

On se réfère au schéma de la figure 1 où il apparaît que le dispositif suivant l'invention comprend essentiellement une alimentation à découpage 1 commandée par un compteur 2 lui-même connecté à des moyens de mémoire et de chargement de nombres constitués par un microprocesseur 3, par exemple. L'alimentation à découpage est classiquement constituée par une inductance $L_1$ disposée en série avec un transistor $T_1$ du type MOS canal P, entre une source de tension de référence

$V_{cc}$ et une ligne de masse 4. Une diode $D_1$ est montée passante entre le point commun à l'inductance $L_1$ et au drain du transistor $T_1$ d'une part et une borne d'une capacité $C_1$ d'autre part, disposée entre la diode et la ligne de masse 4. Le fonctionnement d'une telle alimentation à découpage 1 est classique. Le courant dans l'inductance $L_1$ croît à chaque fois que le transistor $T_1$ est conducteur, avec une pente qui est déterminée par la valeur de l'inductance et celle de la tension $V_{cc}$. Lorsque $T_1$ cesse de conduire, l'énergie accumulée dans le champ magnétique de l'inductance $L_1$ se transfère dans la capacité $C_1$ à travers la diode $D_1$. A chaque transfert la tension entre les bornes 5 et 6 de la capacité $C_1$ s'accroît par pas et peut, en l'absence de charge connectée entre les bornes 5 et 6, atteindre une tension très élevée.

Suivant la présente invention, l'alimentation à découpage 1 est commandée par une sortie "détection de zéro" du compteur 2, qui peut être par exemple un compteur/décompteur (ou régressif) synchrone à huit étages. A chaque fois que le compteur passe par zéro, une impulsion de durée déterminée apparaît sur cette sortie et débloque le transistor $T_1$ qui déclenche la croissance du courant dans l'inductance $L_1$.

Le compteur 2 est lui-même chargé avec un nombre ou "vecteur" défini sur huit bits, par exemple. Le vecteur est délivré sur des entrées de données en parallèle 7 du compteur pour charger une borne supérieure du décomptage exécutée par le compteur. Ainsi l'intervalle de temps séparant l'apparition de deux impulsions successives sur la sortie "détecteur de zéro" est-il fonction de la valeur de ce nombre ou vecteur. En rapprochant ces impulsions dans le temps, on accélère la vitesse de charge de la capacité $C_1$ alors qu'en les écartant on diminue cette vitesse de charge. On conçoit que l'on peut ainsi donner à la tension disponible entre les bornes 5 et 6, débitant sur une charge extérieure fixe (non représentée), une forme quelconque définie par une suite de valeurs d'un "vecteur" chargées successivement dans le compteur pour faire varier l'intervalle de temps séparant deux impulsions consécutives de déblocage du transistor $T_1$.

La suite des valeurs du vecteur correspondant à une forme d'onde donnée, sinusoïdale, triangulaire, en créneau ou en dents de scie par exemple, symétrique ou non, constitue ainsi une suite de nombres dont chacun est une "image" d'un des segments successifs suivant lesquels on peut découper une forme d'onde. Cette suite de nombres est conservée dans des moyens de mémoire qui peuvent, préférentiellement mais non exclusivement, être internes au microprocesseur 3. Celui-ci est dûment programmé pour rythmer la délivrance de ces valeurs au compteur, celui-ci "tournant" constamment entre la valeur maximum chargée et la valeur 0, le comptage reprenant immédiatement à chaque passage par 0 du fait du bouclage de la sortie du détecteur de zéro sur l'entrée de "validation" de l'entrée 7. Classiquement, comme représenté, le compteur est connecté à une horloge 8 et alimenté entre la source de tension

$V_{cc}$ et la ligne de masse 4.

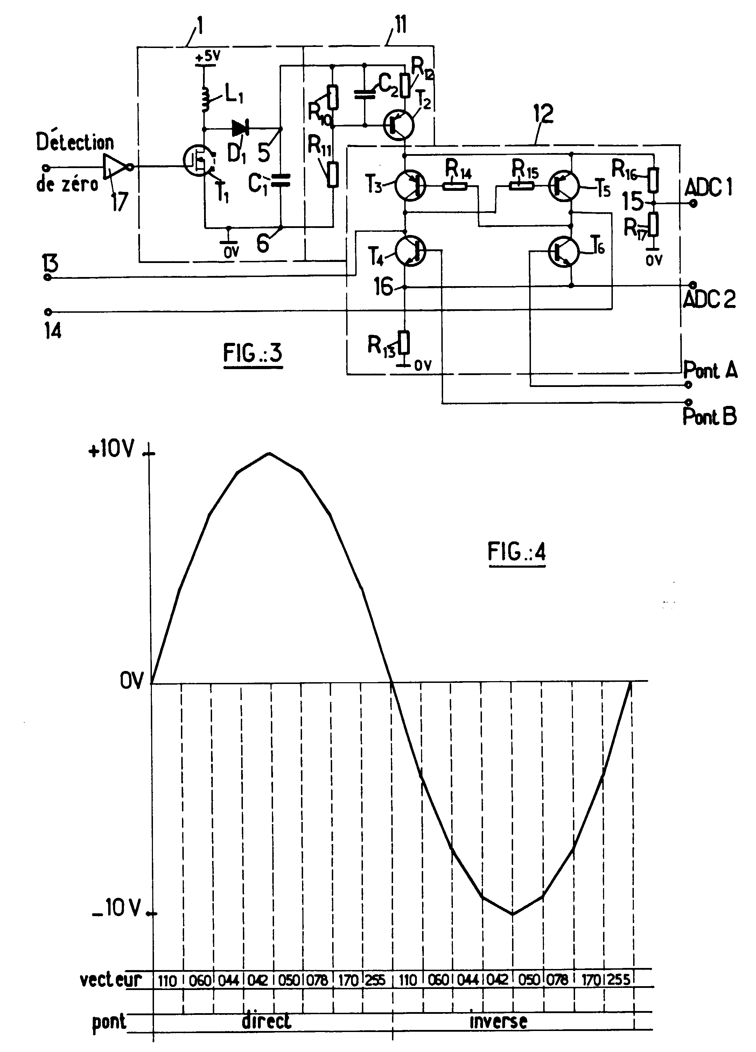

On a représenté à titre d'exemple sur la figure 4 la suite des valeurs du vecteur utilisable pour obtenir une forme d'onde sinusoïdale, en supposant toujours que l'alimentation débite sur une charge extérieure fixe. Une valeur élevée du vecteur (110 par exemple) a pour effet d'établir une période de comptage longue et par conséquent une fréquence d'excitation faible de l'alimentation à découpage, qui résulte en une tension de sortie faible. Un vecteur de plus faible valeur assure une période de comptage plus courte, une plus grande fréquence d'excitation de l'alimentation à découpage et donc une tension de sortie accrue. En faisant varier le vecteur rapidement de manière cyclique, on peut engendrer n'importe quelle forme d'onde y compris des niveaux constants, des impulsions triangulaires ou rectangulaires, des dents de scie et des sinusoïdes. Pour la génération d'une forme d'onde alternative symétrique et sinusoïdale telle que celle représentée à la figure 4, on peut engendrer la deuxième demi-onde de la forme d'onde par simple inversion du sens de passage du courant dans la charge extérieure, obtenue par des moyens que l'on décrira plus loin, la séquence des valeurs du vecteur (110, 060, 044, etc ...) pour une demi-onde seulement étant alors mise en mémoire.

Suivant une caractéristique avantageuse du dispositif selon l'invention, celui-ci comprend des moyens particulièrement simples pour faire varier la fréquence de la forme d'onde établie : il suffit de régler la cadence avec laquelle le microprocesseur charge le compteur avec les valeurs successives du vecteur.

On remarquera que la durée de l'impulsion de déblocage du transistor $T_1$ est égale à la période du signal délivré par l'horloge 8 qui rythme le compteur 2, cette durée étant ainsi fixée avec une grande précision. La période de répétition de l'impulsion est variable, égale à la durée de l'impulsion de base multipliée par la valeur du vecteur, augmentée d'une unité. On remarquera encore que l'alimentation à découpage 1 est du type qui permet de délivrer une tension de sortie supérieure à la tension d'entrée. Ceci est nécessaire dans l'application décrite plus loin de l'invention à un appareil ionophorétique d'administration transdermique de médicaments, qui doit être alimenté par de petites piles pour être portable sans gêne par un patient. Dans une telle application, il faut pouvoir atteindre, comme on l'a vu plus haut, des tensions supérieures à 10 V avec deux piles de 3 V par exemple, et il faut donc disposer d'une alimentation à découpage élévatrice de tension.

On se réfère maintenant à la figure 2 du dessin annexé où l'on a représenté en plus de détail les moyens utilisés pour charger une valeur de vecteur dans le compteur 2, à partir du microprocesseur 3. A titre d'exemple non limitatif, le compteur 2 est constitué par un compteur régressif synchrone à huit étages référencé 74HC40103 dans les catalogues de la Société SGS THOMSON alors que le microprocesseur utilisé est celui référencé M50927E dans les catalogues de la Société MITSUBISHI.

On a représenté chacune des huit lignes de l'entrée parallèle 7 du compteur, chargée chacune par une résistance ($R_2$ à $R_9$), comme cela est classique. Les sorties $S_0$ à $S_3$ et $P_0$ à $P_3$ du microprocesseur alimentent ces lignes, raccordées aux broches 4 à 13 du compteur. Une horloge à cristal 8 alimente le compteur à travers un inverseur 9, et le microprocesseur directement sur son entrée d'horloge XIN. La remise à zéro du microprocesseur et du compteur peut être commandée par une borne RAZ. Une source de tension de référence 10 alimente le microprocesseur. Du côté du compteur 2, la sortie "détection de zéro" est bouclée sur une entrée de validation EN2, comme sur le schéma de la figure 1.

On voit apparaître encore sur la figure 2, deux lignes "pont A", "pont B" connectées à des sorties $D_3$ et $D_4$ respectivement du microprocesseur et deux lignes ADC1 et ADC2 connectées à des entrées K0 et K1 du microprocesseur qui sont munies de convertisseurs analogique/numérique intégrés. Les rôles de ces quatres lignes seront explicités en liaison avec l'examen qui va suivre du schéma représenté à la figure 3.

Sur cette figure on a entouré de traits interrompus trois blocs correspondant respectivement à l'alimentation à découpage 1 décrite ci-dessus, à des moyens de stabilisation de courant 11 et à des moyens 12 d'inversion du sens de courant dans la charge extérieure. La base du transistor $T_1$ de l'alimentation à découpage 1 est connectée à la sortie "détection de zéro" du compteur 2, à travers un inverseur 17.

Les deux lignes ADC1 et ADC2 précitées font partie de moyens de régulation en boucle fermée du courant ou de la tension appliquée à cette charge extérieure qui est connectée entre des électrodes 13 et 14 formant partie, dans une application particulière de l'invention, d'un appareil ionophorétique d'administation transdermique de médicaments.

On constitue un tel appareil en disposant dans un boîtier susceptible d'être maintenu par un bracelet sur un membre d'un patient, un dispositif de génération d'une tension électrique suivant la présente invention et une ou plusieurs piles électriques de faible encombrement telles que de piles au lithium pour l'alimentation du dispositif. Les électrodes 13,14 sont connectées, de manière éventuellement séparable, aux bornes de sortie du circuit de la figure 3. Au moins une de ces électrodes est garnie d'une réserve du médicament à administrer, tel qu'un hydrogel chargé de la forme ionisée de ce médicament, cet hydrogel étant appliqué contre la peau d'un patient à traiter. Les deux électrodes font alors face à deux plages voisines de la peau du patient et la tension développée par le dispositif suivant l'invention provoque, quand elle est convenablement orientée, une migration des molécules ionisées du médicament à travers la peau du patient, migration forcée par le champ électrique établi entre les deux électrodes, qui s'enfonce sous la peau du patient.

Jusqu'à présent, on a décrit l'invention dans son application à une migration ionophorétique de médicaments. Il est clair, bien entendu, que l'invention s'étend à toute administration transdermique de médicaments véhiculés électriquement à travers la peau, que ce soit par ionophorèse ou par électro-osmose, par exemple, ou par une combinaison de ces deux effets.

Le circuit de la figure 3 est conçu pour assurer diverses fonctions particulièrement utiles dans l'application décrite ci-dessus.

C'est ainsi que ce circuit comprend les moyens de stabilisation du courant 11 délivré au patient, ces moyens comprenant un transistor $T_2$ dont la base est reliée au point milieu d'un pont de résistance $R_{10},R_{11}$ connecté aux bornes de la capacité $C_1$, une résistance $R_{12}$ disposée sur l'émetteur du transistor $T_2$ limitant le courant délivré par ce transistor à la charge extérieure. En effet, la base du transistor $T_2$ est maintenue à une tension proportionnelle à celle de la borne 5 de la capacité $C_1$ grâce au pont $R_{10},R_{11}$. La tension aux bornes de la résistance d'émetteur $R_{12}$ est alors proportionnelle à celle régnant sur la borne 5 diminuée d'une valeur constante déterminée par la tension émetteur/base du transistor $T_2$. La tension aux bornes de la résistance d'émetteur $R_{12}$ détermine à son tour le courant d'émetteur et, de là, le courant dans le collecteur de ce transistor. Ce courant est sensiblement indépendant de la résistance de collecteur qui est partiellement déterminée par celle du patient, telle qu'elle est vue entre les électrodes 13 et 14. Les moyens de stabilisation décrits rendent l'intensité du courant circulant dans le patient (et donc le débit de médicament) sensiblement indépendant de la résistance du patient, qui peut varier d'un sujet à l'autre. On notera encore la présence d'une capacité $C_2$ connectée en parallèle sur la résistance $R_{10}$, l'ensemble constituant un filtre qui lisse la sortie de l'alimentation à découpage 1.

Comme on l'a vu plus haut, le dispositif suivant l'invention comprend aussi des moyens 12 pour inverser le sens du courant dans la charge extérieure, entre les deux électrodes 13,14, ce qui permet d'engendrer des formes d'ondes alternatives constituées de deux demi-ondes symétriques à l'aide de la mémorisation des valeurs de vecteur correspondant à une demi-onde seulement. Sur la figure 3 il apparaît que ces moyens 12 sont interposés entre le transistor $T_2$ et les électrodes 13,14. Les moyens 12 sont constitués par un pont de quatre transistors $T_3,T_4,T_5,T_6$ agencés de manière à alimenter la charge extérieure par une diagonale, les bases de deux transistors $T_3,T_5$ d'un premier type (PNP) étant couplées aux collecteurs des deux autres transistors $T_4,T_6$ d'un deuxième type (NPN) dont les états de conduction sont inversés à la détection par les moyens de chargement d'un nombre correspondant au passage par zéro de la forme d'onde.

Bien entendu, le pont de transistors pourrait aussi être utilisé pour engendrer des formes d'onde présentant des alternances dissymétriques, l'ensemble de la forme d'onde devant alors être mémorisé.

Les bases des transistors $T_3$,$T_5$ sont raccordées au collecteur des transistors $T_4$,$T_6$ respectivement à travers des résistances $R_{14}$,$R_{15}$ respectivement. Les bases des transistors $T_4$,$T_6$ sont commandées par des signaux "pont A", "pont B" respectivement émis par le microprocesseur 3 (voir figure 2). Les électrodes 13,14 sont connectées aux collecteurs des transistors $T_3$ et $T_5$, suivant une "diagonale" du pont. Dans le mode de réalisation représenté, les moyens de mémoire et les moyens de chargement des valeurs du vecteur sont réunis dans le microprocesseur et celui-ci est en outre muni de moyens de détection de la valeur du vecteur qui correspond au passage par zéro de la forme d'onde, ces moyens déclenchant l'émission des signaux "pont A" et "pont B". Il est clair que, lorsque le transistor $T_4$ est rendu conducteur par l'émission d'un signal pont A, la conduction du transistor $T_4$ déclenche celle de $T_5$, le courant sortant du collecteur du transistor $T_2$ passant dans le patient de l'électrode 14 vers l'électrode 13. Le courant passe dans l'autre sens quand le microprocesseur déclenche la conduction des transistors $T_3$ et $T_6$.

Dans l'application d'un tel dispositif à une migration ionophorétique de médicaments, la migration à double sens que l'on peut ainsi obtenir est avantageuse lorsqu'on utilise deux électrodes associées chacune à une réserve de médicament. Par rapport à un appareil comprenant une seule réserve associée à une des électrodes seulement, on peut doubler le débit de médicament. Pour un même débit, on établit celui-ci à partir de deux plages distinctes de la peau du patient, ce qui permet de réduire l'irritation de celle-ci due à l'effet transdermique.

Suivant une autre caractéristique avantageuse du dispositif selon l'invention, celui-ci peut être muni de moyens permettant d'assurer une régulation en boucle fermée de la tension ou de l'intensité du courant appliquées au patient entre les électrodes 13 et 14. La résistance du patient peut en effet varier dans le temps, au cours du traitement, en particulier du fait de la polarisation de la couche cornée de la peau, ceci malgré l'effet dépolarisant de l'application de tensions pulsées qui limitent cette polarisation. Cette variation de résistance peut entraîner des variations de la tension ou du courant appliqué au patient, hors de domaines prédéterminés en dehors desquels le traitement n'est pas correctement exécuté.

Suivant l'invention, on détecte de telles variations de résistance à l'aide de tensions $V_1$,$V_2$, prélevées en amont et en aval du pont de transistor $T_3$,$T_4$,$T_5$,$T_6$, aux points 15 et 16 respectivement, par les lignes ADC1 et ADC2, respectivement.

La tension $V_1$ prélevée au point milieu 15 d'un pont diviseur $R_{16}$,$R_{17}$ connecté entre les collecteurs des transistors $T_2$,$T_5$ et la masse est fournie à l'entrée K0 du microprocesseur alors que la tension $V_2$ sur les émetteurs des transistors $T_4$,$T_6$ est délivrée à l'entrée K1 du microprocesseur, qui les convertit en valeurs numériques.

La tension $V_1$ permet au microprocesseur de calculer la tension sur l'électrode d'entrée du courant passant dans le patient alors que la tension $V_2$ appliquée à la résistance $R_{13}$ placée entre les émetteurs des transistors $T_4$,$T_6$ et la masse permet de calculer le courant $i = V_2/R_{13}$ passant dans le patient. Le microprocesseur calcule ensuite la résistance R du patient :

$$R = (kV_1 - V_2)/i ,$$

k étant égal à

$$\frac{R_{16} + R_{17}}{R_{17}}$$

A partir des variations de la résistance du patient qu'il peut ainsi observer, le microprocesseur réalise une commande en boucle fermée de la tension ou du courant appliqué au patient en accroissant ou en décroissant la valeur du vecteur qui est fournie au compteur de manière à maintenir ainsi la valeur désirée de tension ou d'intensité de courant. La contre-réaction ainsi obtenue peut également permettre de commander un arrêt ou une modification de la stimulation du patient en cas de tension, d'intensité ou de résistance excessive ou inadéquate, mesurée sur le patient.

On a construit un appareil ionophorétique d'administration transdermique de médicament comprenant un dispositif de génération de tension conforme à la présente invention. Alimenté par deux piles au lithium du type 2430, l'appareil permet de délivrer au patient un courant réglable entre 0 et 1 mA, en continu ou suivant des impulsions de forme d'onde quelconque, éventuellement bipolaires et de fréquence jusqu'à 80 Hz pour une forme d'onde sinusoïdale ou triangulaire et jusqu'à 2 kHz pour des formes d'onde carrées, la précision du courant obtenu étant meilleure que 0,05 mA, ceci pour des résistances de peau allant jusqu'à 20 kΩ. Ces résultats ont pu être obtenus grâce aux caractéristiques et avantages de la présente invention exposés ci-dessus et notamment grâce à la technique de chargement en série des valeurs du "vecteur" dans le compteur qui permet de configurer avec une grande souplesse une forme d'onde quelconque. La régulation en boucle fermée par logiciel incorporé au microprocesseur et la mesure précise de la résistance du patient permettent de régler le courant passant dans le patient avec une grande précision, ce qui compense des pertes éventuelles dans le circuit. L'utilisation d'une alimentation à découpage élévatrice de tension, commandée par un compteur, permet de disposer d'une source de tension précise et de faible encombrement. L'utilisation suivant l'invention d'une forme d'onde bipolaire en combinaison avec deux électrodes garnies chacune d'un hydrogel chargé de médicaments à administrer au patient, permet d'assurer cette administration dans les deux sens de circulation du courant, à travers deux plages différentes de la peau du patient, technique qui accroît la tolérance du patient au courant ionophorétique.

Bien entendu l'invention n'est pas limitée au mode de réalisation décrit et représenté, qui n'a été donné qu'à titre d'exemple. C'est ainsi que l'on peut remplacer le compteur régressif utilisé par un compteur progressif, moyennant des adaptations qui viennent immédiatement à l'esprit de l'homme de métier.

## Revendications

1. Dispositif de génération d'une tension électrique de forme d'onde prédéterminée, comprenant une alimentation à découpage (1) munie d'un organe de commutation électronique ($T_1$) dont la fermeture commande l'alimentation d'une inductance ($L_1$) qui se décharge, à la réouverture de l'organe, dans une capacité ($C_1$) aux bornes de laquelle apparaît la tension de sortie du dispositif, caractérisé en ce qu'il comprend (a) des moyens de mémoire pour enregistrer une suite de nombres qui sont des images de segments successifs de la forme d'onde prédéterminée, (b) un compteur numérique cadencé (2), et (c) des moyens (3) pour charger successivement ce compteur (2) avec chacun des nombres de la suite en tant que borne du comptage opéré par le compteur (2), celui-ci commandant cycliquement la fermeture de l'organe de commutation ($T_1$) pendant un intervalle de temps prédéterminé, à chaque fois que le comptage opéré atteint la borne ainsi fixée.

2. Dispositif conforme à la revendication 1, caractérisé en ce que le compteur est un compteur régressif (2) décomptant à partir du nombre chargé et muni d'une sortie de détection de zéro commandant la fermeture de l'organe de commutation pendant l'intervalle de temps prédéterminé, cet organe étant constitué par un transistor ($T_1$).

3. Dispositif conforme à la revendication 2, caractérisé en ce que la sortie de détection de zéro du compteur (2) est bouclée sur une entrée de validation du compteur, pour déclencher un nouveau décomptage à partir du nombre chargé.

4. Dispositif conforme à l'une quelconque des revendications 1 à 3, caractérisé en ce que les moyens pour charger successivement le compteur avec chacun des nombres de la suite sont constitués par un microprocesseur (3).

5. Dispositif conforme à la revendication 4, caractérisé en ce que les moyens de mémoire sont programmables suivant diverses suites de nombres pour l'obtention d'autant de formes d'onde prédéterminées distinctes.

6. Dispositif conforme à la revendication 4, caractérisé en ce que les moyens de mémoire sont incorporés au microprocesseur (3).

7. Dispositif conforme à l'une quelconque des revendications 1 à 6, caractérisé en ce qu'il comprend en outre des moyens de stabilisation de courant (11) interposés entre la capacité ($C_1$) et une charge extérieure alimentée par la tension délivrée par le dispositif.

8. Dispositif conforme à la revendication 7, caractérisé en ce que lesdits moyens de stabilisation comprennent un transistor ($T_2$) dont la base est reliée au point milieu d'un pont de résistances ($R_{10}, R_{11}$) connecté aux bornes de la capacité ($C_1$), une résistance ($R_{12}$) limitant le courant délivré par le transistor ($T_2$) à la charge extérieure.

9. Dispositif conforme à l'une quelconque des revendications 1 à 8, caractérisé en ce qu'il comprend en outre des moyens (12) pour inverser le sens de circulation dans une charge extérieure, du courant de sortie du dispositif, ces moyens étant activés à la détection du chargement dans le compteur (2) d'un nombre image d'un passage à zéro de la forme d'onde souhaitée.

10. Dispositif conforme à la revendication 9, caractérisé en ce que les moyens de mémoire sont chargés avec les nombres images d'une demi-onde seulement d'une forme d'onde alternative à deux demi-ondes symétriques.

11. Dispositif conforme à l'une quelconque des revendications 9 et 10, caractérisé en ce que lesdits moyens d'inversion (12) sont constitués par un pont de quatre transistors ($T_3, T_4, T_5, T_6$) agencés de manière à alimenter une charge extérieure par une diagonale, les bases de deux transistors ($T_3, T_5$) d'un premier type étant couplées aux collecteurs des deux autres transistors ($T_4, T_6$) d'un deuxième type dont les états de conduction sont inversés à la détection, par les moyens de chargement, d'un nombre correspondant au passage par zéro de la forme d'onde.

12. Dispositif conforme à l'une quelconque des revendications précédentes, caractérisé en ce que les moyens de chargement comprennent des moyens pour faire varier la fréquence de chargement des nombres de la suite et ainsi la fréquence de la tension de sortie du dispositif.

13. Dispositif conforme à l'une quelconque des revendications 4 à 12, caractérisé en ce qu'il comprend des moyens de régulation en boucle fermée de la tension ou de l'intensité du courant de sortie du dispositif.

14. Appareil d'administration transdermique de médicaments, du type comprenant au moins deux électrodes mises en contact avec deux plages voisines de la peau d'un patient, une réserve d'un médicament à molécules véhiculées électriquement étant disposée contre au moins une des électrodes pour venir en contact avec la peau du patient, caractérisé en ce que les électrodes sont alimentées par la sortie du dispositif conforme à l'une quelconque des revendications 1 à 13.

15. Appareil conforme à la revendication 14, alimenté par un dispositif conforme à l'une quelconque des revendications 9 à 11, caractérisé en ce qu'une réserve de médicaments est disposée contre chacune des deux électrodes.

16. Appareil conforme à l'une quelconque des revendications 14 et 15, alimenté par un dispositif conforme à la revendication 13, caractérisé en ce qu'il comprend des moyens pour mesurer la tension et le courant circulant dans le patient entre les deux électrodes de manière à calculer la résistance du patient, vue entre ces deux électrodes, et des moyens de correction des nombres chargés successivement dans le compteur (2) pour compenser l'influence d'une variation de cette résistance sur la tension ou le courant circulant dans le patient.

17. Appareil conforme à la revendication 16, caractérisé en ce que les moyens de calcul et de correction sont incorporés au microprocesseur (3).

**Patentansprüche**

1. Vorrichtung zur Erzeugung einer elektrischen Spannung mit vorbestimmter Wellenform, umfassend eine Zerhackungsversorgung (1), die mit einem elektronischen Schaltorgan ($T_1$) versehen ist, dessen Schließung die Versorgung einer Induktanz ($L_1$) auslöst, die sich bei der Wiederöffnung des Organs in eine Kapazität ($C_1$) entlädt, an deren Anschlüssen die Ausgangsspannung der Vorrichtung auftritt, dadurch gekennzeichnet, daß sie (a) Speichermittel zum Aufzeichnen einer Folge von Zahlen, die Abbilder von aufeinanderfolgenden Segmenten der vorbestimmten Wellenform sind, (b) einen getakteten digitalen Zähler (2) und (c) Mittel zum aufeinanderfolgenden Laden dieses Zählers (2) mit jeder der Zahlen der Folge als Endwert der von dem Zähler (2) vorgenommenen Zählung umfaßt, wobei dieser zyklisch jedesmal, wenn die vorgenommene Zählung den so festgelegten Endwert erreicht, die Schließung des Schaltorgans ($T_1$) während eines vorbestimmten Zeitintervalls auslöst.

2. Vorrichtung nach Anspruch 1, dadurch gekennzeichnet, daß der Zähler ein regressiver Zähler (2) ist, der von der geladenen Zahl zurückzählt und mit einem Nullerfassungsausgang versehen ist, der die Schließung des Schaltorgans während des vorbestimmten Zeitintervalls auslöst, wobei dieses Organ aus einem Transistor ($T_1$) besteht.

3. Vorrichtung nach Anspruch 2, dadurch gekennzeichnet, daß der Nullerfassungsausgang des Zählers (2) zu einem Validierungseingang des Zählers geschleift ist, um eine neue Zurückzählung von der geladenen Zahl aus auszulösen.

4. Vorrichtung nach einem der Ansprüche 1 bis 3, dadurch gekennzeichnet, daß die Mittel zum aufeinanderfolgenden Laden des Zählers mit jeder der Zahlen der Folge aus einem Mikroprozessor (3) bestehen.

5. Vorrichtung nach Anspruch 4, dadurch gekennzeichnet, daß die Speichermittel gemäß verschiedenen Folgen von Zahlen programmierbar sind, um ebensoviele verschiedenen vorbestimmte Wellenformen zu erhalten.

6. Vorrichtung nach Anspruch 4, dadurch gekennzeichnet, daß die Speichermittel in den Mikroprozessor (3) eingegliedert sind.

7. Vorrichtung nach einem der Ansprüche 1 bis 6, dadurch gekennzeichnet, daß sie außerdem Stromstabilisierungsmittel (11) aufweist, die zwischen die Kapazität ($C_1$) und eine äußere Last eingesetzt sind, die mit der von der Vorrichtung gelieferten Spannung versorgt wird.

8. Vorrichtung nach Anspruch 7, dadurch gekennzeichnet, daß diese Stabilisierungsmittel einen Transistor ($T_2$) umfassen, dessen Basis mit dem Mittelpunkt einer Widerstandsbrücke ($R_{10}$, $R_{11}$) verbunden ist, die an die Anschlüsse der Kapazität ($C_1$) angeschlossen ist, wobei ein Widerstand ($R_{12}$) den vom Transistor ($T_2$) der äußeren Last gelieferten Strom begrenzt.

9. Vorrichtung nach einem der Ansprüche 1 bis 8, dadurch gekennzeichnet, daß sie außerdem Mittel (12) zum Umkehren der Strömungsrichtung des Ausgangsstroms der Vorrichtung in einer äußeren Last umfaßt, die bei der Erfassung der Ladung einer Zahl, die Abbild eines Nulldurchgangs der gewünschten Wellenform ist, in den Zähler (2) aktiviert werden.

10. Vorrichtung nach Anspruch 9, dadurch gekennzeichnet, daß die Speichermittel mit den Zahlen geladen sind, die Abbilder nur einer Halbwelle einer Wechselwellenform mit zwei symmetrischen Halbwellen sind.

**11.** Vorrichtung nach einem der Ansprüche 9 und 10, dadurch gekennzeichnet, daß diese Umkehrmittel (12) aus einer Brücke von vier Transistoren ($T_3$, $T_4$, $T_5$, $T_6$) bestehen, die so angeordnet sind, daß sie eine äußere Last durch eine Diagonale versorgen, wobei die Basen von zwei Transistoren ($T_3$, $T_5$) eines ersten Typs mit den Kollektoren der beiden anderen Transistoren ($T_4$, $T_6$) eines zweiten Typs gekoppelt sind, deren Leitungszustände umgekehrt werden, wenn die Lademittel eine Zahl erfassen, die dem Nulldurchgang der Wellenform entspricht.

**12.** Vorrichtung nach einem der vorhergehenden Ansprüche, dadurch gekennzeichnet, daß die Lademittel Mittel zum Ändern der Ladefrequenz der Zahlen der Folge und so der Frequenz der Ausgangsspannung der Vorrichtung aufweisen.

**13.** Vorrichtung nach einem der Ansprüche 4 bis 12, dadurch gekennzeichnet, daß sie Mittel zur Regelung der Spannung oder der Stärke des Ausgangsstroms der Vorrichtung im geschlossenen Kreis besitzt.

**14.** Gerät zur transdermalen Verabreichung von Medikamenten, umfassend mindestens zwei Elektroden, die mit zwei benachbarten Bereichen der Haut eines Patienten in Kontakt gebracht werden, wobei ein Vorrat an einem Medikament aus elektrisch beförderten Molekülen an mindestens einer der Elektroden angeordnet ist, um mit der Haut des Patienten in Kontakt zu kommen, dadurch gekennzeichnet, daß die Elektroden durch den Ausgang der Vorrichtung nach einem der Ansprüche 1 bis 13 versorgt sind.

**15.** Gerät nach Anspruch 14, das durch eine Vorrrichtung nach einem der Ansprüche 9 bis 11 versorgt ist, dadurch gekennzeichnet, daß ein Medikamentenvorrat an jeder der beiden Elektroden angeordnet ist.

**16.** Gerät nach einem der Ansprüche 14 und 15, das durch eine Vorrichtung nach Anspruch 13 versorgt ist, dadurch gekennzeichnet, daß es Mittel zum Messen der Spannung und des Stroms besitzt, der in dem Patienten zwischen den beiden Elektroden fließt, so daß der zwischen diesen beiden Elektroden gesehene Widerstand des Patienten gemessen wird, und Mittel zur Korrektur der nacheinander in den Zähler (2) geladenen Zahlen, um den Einfluß einer Änderung dieses Widerstands auf die Spannung oder den Strom, der in dem Patient fließt, zu kompensieren.

**17.** Gerät nach Anspruch 16, dadurch gekennzeichnet, daß die Rechen- und Korrekturmittel in den Mikroprozessor (3) eingegliedert sind.

**Claims**

**1.** Device for generating an electrical voltage of predetermined waveform, comprising a switched-mode power supply (1) equipped with an electronic switching member ($T_1$), the closing of which controls the power supply to an inductor ($L_1$) which discharges, when the member reopens, into a capacitor ($C_1$) at the terminals of which the output voltage of the device appears, characterised in that it comprises (a) storage means for recording a sequence of numbers which are images of successive segments of the predetermined waveform, (b) a clocked digital counter (2) and (c) means (3) for successively loading this counter (2) with each of the numbers of the sequence considered as a bound of the count performed by the counter (2), the latter cyclically controlling the closing of the switching member ($T_1$) for a predetermined time interval, each time the count performed reaches the bound thus fixed.

**2.** Device according to Claim 1, characterised in that the counter is a downward counter (2) counting down from the number loaded and equipped with a zero-detection output controlling the closing of the switching member for the predetermined time interval, this member being constituted by a transistor ($T_1$).

**3.** Device according to Claim 2, characterised in that the zero-detection output of the counter (2) is looped back to an enable input of the counter, in order to trigger a new down-count from the number loaded.

**4.** Device according to anyone of Claims 1 to 3, characterised in that the means for successively loading the counter with each of the numbers of the sequence are constituted by a microprocessor (3).

**5.** Device according to Claim 4, characterised in that the storage means are programmable according to various sequences of numbers for obtaining as many distinct predetermined wave forms.

**6.** Device according to Claim 4, characterised in that the storage means are incorporated into the microprocessor (3).

**7.** Device according to anyone of Claims 1 to 6, characterised in that it furthermore comprises current-stabilizing means (11) interposed between the capacitor ($C_1$) and an external load supplied by the voltage delivered by the device.

**8.** Device according to Claim 7, characterised in that the said stabilizing means comprise a transistor

($T_2$), the base of which is connected to the mid point of a resistor bridge ($R_{10}$, $R_{11}$) connected to the terminals of the capacitor ($C_1$), a resistor ($R_{12}$) limiting the current delivered by the transistor ($T_2$) to the external load.

9. Device according to anyone of Claims 1 to 8, characterised in that it comprises means (12) for reversing the direction of flow, into an external load, of the output current of the device, these means being actuated on detecting the loading into the counter (2) of an image number of a zero crossing by the desired waveform.

10. Device according to Claim 9, characterised in that, in the case of an alternating waveform having two symmetrical half-waves, the storage means are loaded with the image numbers of one half-wave only.

11. Device according to anyone of Claims 9 or 10, characterised in that the said reversing means (12) are constituted by a bridge of four transistors ($T_3$, $T_4$, $T_5$, $T_6$) arranged so as to supply an external load by a diagonal, the bases of two transistors ($T_3$, $T_5$) of a first type being coupled to the collectors of the other two transistors ($T_4$, $T_6$) of a second type, the conduction states of which are reversed on detecting, by the loading means, a number corresponding to the zero crossing by the waveform.

12. Device according to anyone of the preceding claims, characterised in that the loading means comprise means for varying the loading frequency of the numbers of the sequence and thus the frequency of the output voltage of the device.

13. Device according to anyone of Claims 4 to 12, characterised in that it comprises means for closed-loop regulation of the voltage or the strength of the output current of the device.

14. Apparatus for transdermally delivering medicinal products, of the type comprising at least two electrodes designed to be put in contact with two adjacent areas of the skin of a patient, a reserve of a medicinal product having electrically transportable molecules being arranged against at least one of the electrodes in order to come into contact with the patient's skin, characterised in that the electrodes are supplied by the output of the device according to anyone of Claims 1 to 13.

15. Apparatus according to Claim 14, supplied by a device according to anyone of Claims 9 to 11, characterised in that a reserve of medicinal products is arranged against each of the two electrodes.

16. Apparatus according to anyone of Claims 14 and 15, supplied by a device according to Claim 13, characterised in that it comprises means for measuring the voltage and the current flowing into the patient between the two electrodes so as to calculate the patient's resistance, seen between these two electrodes, and means for correcting the numbers successively loaded into the counter (2) in order to compensate for the influence of a variation in this resistance on the voltage or the current flowing into the patient.

17. Apparatus according to Claim 16, characterised in that the calculating and correcting means are incorporated into the microprocessor (3).

FIG.:1

FIG.:2

FIG.:3

FIG.:4

| vecteur | 110 | 060 | 044 | 042 | 050 | 078 | 170 | 255 | 110 | 060 | 044 | 042 | 050 | 078 | 170 | 255 |
|---|---|---|---|---|---|---|---|---|---|---|---|---|---|---|---|---|
| pont | direct | | | | | | | | inverse | | | | | | | |